# EUROPEAN PATENT APPLICATION

(11) **EP 2 792 753 A1**
(43) Date of publication of application: **22.10.2014**
(21) Application number: 12858582.5
(22) Date of filing: 14.12.2012
(51) Int. Cl.: C12Q 1/68, C12N 15/12

(54) **METHOD FOR OBTAINING DATA THAT CAN BE USED FOR THE DIAGNOSIS AND PROGNOSIS OF NEUROSENSORY HYPOACUSIS**

(30) Priority: 14.12.2011 ES 201132013
(71) Applicant: Fundación Pública Andaluza Progreso Y Salud, 41092 Sevilla (ES); Servicio Andaluz de Salud, 41071 Sevilla (ES); Agencia Pública Empresarial Sanitaria Hospital de Poniente, 04700 El Ejido (Almería) (ES); Servizo Galego De Saúde (Sergas), 15703 Santiago de Compostela (A Coruña) (ES); Fundación para la Investigación del Hospital Universitario La Fe, 46009 Valencia (ES)
(72) Inventor: LÓPEZ ESCAMEZ, Jose Antonio, E-04700 El Ejido (Almería) (ES); LÓPEZ NEVOT, Miguel Ángel, E-18014 Granada (ES); GAZQUEZ PÉREZ, Irene, E-41092 Sevilla (ES); MORENO CASARES, Antonia María, E-18014 Granada (ES); LÓPEZ NEVOT, Alicia, E-18014 Granada (ES); REQUENA NAVARRO, María Teresa, E-41092 Sevilla (ES); ARAN GONZÁLEZ, Ismael, E-15703 Santiago de Compostela (ES); SOTO VARELA, Andrés, E-15703 Santiago de Compostela (ES); SANTOS PÉREZ, Sofía, E-15703 Santiago de Compostela (ES); PÉREZ GARRIGUES, Herminio, E-46009 Valencia (ES)
(74) Representative: HOFFMANN EITLE
(86) International application number: PCT/ES2012/070869
(87) International publication number: WO 2013/087968

(57) **Abstract**

The invention relates to: a method for obtaining data that can be used to determine an individual's risk of suffering from severe neurosensory hypoacusis, preferably in Ménière's disease, primers that can be used in the determination, and a kit comprising same.

## Description

The present invention is comprised within the field of biomedicine and biotechnology and relates to a method for obtaining useful data for the diagnosis, prognosis and classification of patients suffering from sensorineural hearing loss, and particularly of those patients with Ménière's disease.

### PRIOR STATE OF THE ART

Ménière's disease (MD) is a chronic disorder affecting the inner ear characterized by recurrent episodes of spontaneous vertigo, pressure in the ears, tinnitus and hearing loss. An autoimmune etiology has been proposed for this disease given the increase in the number of autoantibodies observed and the clinical improvement with steroid therapy observed in some patients. The increase in serum circulating immune complexes (CICs) in MD patients suggests that such complexes may be involved in the physiopathology of the disease either as a direct cause of the damage or as a product of an autoimmune anomaly.

Human major histocompatibility complex class I and class II molecules have been described as genetic markers of many autoimmune diseases. In turn, MD has been associated with HLA-B * 27, HLA-B * 44, HLA-B * 13, HLA-Cw * 07, HLA-DBR1 * 1602 and HLA-DRB1 * 1101 in different populations. The MHC class I polypeptide-related sequence A (MICA) encodes a stress-inducible MICA molecule with three extracellular domains, i.e., α1 (encoded by exon 2), α2 (encoded by exon 3) and α3 (encoded by exon 4), a transmembrane (TM) segment (encoded by exon 5) and a carboxy-terminal cytoplasmic tail (encoded by exon 6).

The MICA gene shows a significant degree of nucleotide variation in exons 2, 3 and 4 and carries a short tandem repeat polymorphism (GCT)n (four, five, six or nine, abbreviated as MICA-STR) in exon 5. Furthermore, the MICA * A5.1 allele has a nucleotide (G) insertion between the second and the third triplet repeats, resulting in a premature stop codon.

MIC genes are expressed primarily in gastrointestinal epithelium, endothelial cells and fibroblasts. However, MIC transcriptions have been observed in all major organs except the brain. MICA acts as a ligand for natural killer (NK) cells, γδ and αβ T-cells, CD8+ T-cells, expressing a common NK cell-activating receptor, NKG2D. MICA-NKG2D interaction seems to increase production of inflammatory cytokines and proliferation of a specific T-cell subset.

Several research studies have suggested an association between the MICA gene and autoimmune diseases. In addition, the MICA gene product may play an important role in the autoimmune process, and MICA gene polymorphisms can modulate immune pathways that are complementary to and synergistic with those modulated by conventional HLA class II genes.

A large variety of medical and surgical treatments for hearing loss, and specifically for Ménière's disease, has been proposed. All these treatments have different results with respect to the remission of symptoms and side effects.

The medical treatments include a low-sodium diet, antihistamines, diuretics, subcutaneous histamine, anti-vertigo drugs, benzodiazepines and transtympanic therapy, which has gained certain popularity in recent times, particularly in that group of patients who do not respond to conventional therapies.

Intratympanic injection of ototoxic medications, such as gentamicin, into the middle ear cavity has been done in some centers for some time. The objective of this procedure is to produce a medical labyrinthectomy which would lead to the decrease or disappearance of symptoms. The risk of this procedure is the deterioration of the patient's hearing ability, particularly described in those patients subjected to short-term treatment regimens. The most widely used agent was initially streptomycin and is now gentamicin.

Surgery has been performed in very serious cases, but this approach impairs the patient's quality of life because it eliminates not only symptoms, but also sense of balance.

Therefore, treatments available today for hearing loss, such as surgery or intratympanic gentamicin application, for example, can be extremely aggressive and can put the patient's hearing at risk. Therefore, it is important to have a marker that allows evaluating treatment suitability.

However, up until now there has been no biological method or marker available that allows early diagnosis or establishing a prognosis with respect to long-term hearing loss that is useful for classifying patients and facilitates decision-making with respect to treatment and monitoring of disease progression.

### DESCRIPTION OF THE INVENTION

The present invention provides a method for obtaining useful data for the early diagnosis and prognosis of hearing loss, as well as for evaluating the response to treatment of said disease, which allows monitoring and establishing groups of patients. The authors of the present invention are the first to demonstrate the association of polymorphic microsatellites, such as MICA-STR, with hearing loss progression in MD, in turn demonstrating that the association with other previous markers lacks statistical support. Therefore, allelic variants of MICA-STR in exon 5 influence susceptibility to and time course of hearing loss in MD, preferably in the Spanish population.

Therefore, a first aspect of the invention relates to the use of the MICA gene for obtaining useful data in the diagnosis and/or prognosis of sensorineural hearing loss.

In this specification, "sensorineural hearing loss" is understood as the decrease in hearing level below what is considered normal, so depending on the intensity loss measured in *decibels* (dB), hearing loss is classified as:
Mild hearing loss - when hearing loss is less than 35 dB.
Moderate hearing loss - when hearing loss is between 35 and 60 dB. Profound or severe hearing loss - when hearing loss is between 60 and 90 dB.
Complete hearing loss or anacusis - when hearing loss is greater than 90

Furthermore, in Ménière's disease, the *American Academy of Otolaryngology-Head and Neck Surgery* (AAO-HNS) defined in 1995 four stages of hearing loss based on the hearing threshold of 500, 1000, 2000 and 3000 Hz frequencies, where hearing loss is classified as:
Stage 1- when hearing loss is less than or equal to 25 dB
Stage 2- when hearing loss is between 26 and 40 dB
Stage 3- when hearing loss is between 41-70 dB
Stage 4- When hearing loss is greater than 70 dB

It is determined by means of pure tone audiometry. Hearing loss can manifest in one or both ears, and it can be sudden or abrupt, rapidly progressive (weeks or months) or slowly progressive (months or years). Sensorineural hearing loss affects the inner ear and occurs when the sensorineural elements of the cochlea or the cochlear nerve are somehow injured by physical means or any other type of means.

The cochlea is the fundamental auditory organ located in the inner ear, and it is a spiral-shaped duct containing the organ of Corti where mechanical vibrations are transformed into nerve impulses reaching the brain through the auditory nerve for identification. When organ of Corti cells and/or the auditory nerve are affected, sound transmission is interrupted. Any of these delicate structures can be affected by different infectious, inflammatory, toxic or degenerative processes, and among the aforementioned, loud noise and degenerative processes are responsible for most cases of perceptive deafness.

Depending on the affected organ, hearing loss can be classified as:
- *Endocochlear hearing loss.* When the inner ear is affected.
- *Retrocochlear hearing loss.* When the auditory nerve is affected.

In a preferred embodiment of this aspect of the invention, the sensorineural hearing loss is selected from the list consisting of: sudden hearing loss, rapidly progressive hearing loss, slowly progressive hearing loss, low-frequency sensorineural hearing loss, immune-mediated inner ear disease, or any combinations thereof. In another preferred embodiment, hearing loss occurs in individuals from Western Europe, and more preferably in the Spanish population or in individuals having the same or a similar gene pool.

In another preferred embodiment, sensorineural hearing loss is furthermore associated with recurrent vertigo attacks. In an even more preferred embodiment, sensorineural hearing loss is associated with Ménière's disease. In another more preferred embodiment, the region of the MICA gene used is the 5' region of the terminal exon (SEQ ID NO: 2).

As understood in this specification, the MICA gene, or MHC class I polypeptide-related sequence A (DAMA-345G11.2, MIC-A, PERB11.1), encodes the MHC (HLA) class I chain-related gene A. The protein product is expressed on the cell surface, although unlike canonical class I molecules, it does not seem to associate with beta-2-microglobulin. MICA's functions as a stress-induced antigen in intestinal epithelial cells are thought to be recognized by gamma delta T lymphocytes and NK cells through the common NKG2D receptor. Alternative splicing gives rise to several transcription variants. It is found in chromosome 6 (6p21.33).

In the context of the present invention, MICA is also defined by a polynucleotide or nucleotide sequence having GenBank (NCBI) accession number NR_036524 and/or in SEQ ID NO: 1, or any variants thereof.

### DIAGNOSTIC METHOD

Another aspect of the invention relates to a method for obtaining useful data for the diagnosis and/or prognosis of sensorineural hearing loss, hereinafter first method of the invention, which comprises:
a) obtaining genomic DNA from a biological sample isolated from an individual, and
b) detecting variants of the MICA gene in the genomic DNA of the biological sample isolated in (a).

In a preferred embodiment, the first method of the invention further comprises:
c) comparing the variants detected in step (b) with a reference variant.

An "isolated biological sample" includes, but is not limited to, cells, tissues and/or biological fluids of an organism obtained by means of any method known by a person skilled in the art. Preferably, the isolated biological sample is peripheral blood and/or comprises peripheral blood cells (PBCs), more preferably mononuclear peripheral blood cells.

In another preferred embodiment of this aspect of the invention, the biological sample isolated from an individual of step (a) is obtained from peripheral blood. In an even more preferred embodiment, the genotype of the individual has the gene pool characteristic of the Western Europe population, and more preferably, characteristic of the Iberian Peninsula and/or the Spanish population.

In a preferred embodiment of this aspect of the invention, the sensorineural hearing loss is selected from the list consisting of: sudden hearing loss, rapidly progressive hearing loss, slowly progressive hearing loss, low-frequency sensorineural hearing loss, immune-mediated inner ear disease, or any combinations thereof.

In another preferred embodiment, the sensorineural hearing loss is furthermore associated with recurrent vertigo attacks. In an even more preferred embodiment, the MICA gene is used for obtaining useful data in the diagnosis and/or prognosis of the risk of suffering from Ménière's disease.

As it is used herein, the term "individual" refers to animals, preferably mammals, and more preferably humans. The term "individual" is not intended to be limiting in any aspect, the individual being able to be of any age, sex and physical condition.

In the sense used in this description, the term "variant" refers to a sequence substantially homologous to the MICA gene sequence. Generally, a variant includes additions, deletions or substitutions of nucleotides (and microsatellite repeats). More preferably, the described variants refer to the number of GCT sequence repeats in exon 5 of the MICA gene.

As it is used herein, a sequence is "substantially homologous" to the sequence having SEQ ID NO: 1 when its sequence presents good alignment with said sequence having SEQ ID NO: 1.

The term "polymorphism" refers to a variation in the nucleotide sequence of a nucleic acid where every possible sequence is present in a proportion equal to or greater than 1% in a population; in a particular case, when said variation in the nucleotide sequence occurs in a single nucleotide (A, C, T or G), it is called an SNP.

The term "gene mutation" refers to a variation in the nucleotide sequence of a nucleic acid where every possible sequence is present in a proportion of less than 1% in a population.

The terms "allelic variant" or "allele" are used interchangeably herein and refer to a polymorphism occurring in one and the same locus in one and the same population.

As it is used herein, the term "gene variation" or "gene variant" includes mutations, polymorphisms and allelic variants. Gene variation or gene variant is found among individuals within populations and among populations within species.

The different alleles or microsatellite sequences of the exon 5 of the MICA gene detected in the example of the invention are:

| | | |
|---|---|---|
| MICA A4 | GCTGCTGCTGCT | SEQ ID NO: 5 |
| MICA A5 | GCTGCTGCTGCTGCT | SEQ ID NO: 6 |
| MICA A5.1 | GCTGCTGGCTGCTGCTG | SEQ ID NO: 7 |
| MICA A6 | GCTGCTGCTGCTGCTGCT | SEQ ID NO: 8 |
| MICA A9 | GCTGCTGCTGCTGCTGCTGCTGCTGCT | SEQ ID NO: 9 |

The variants are preferably the different alleles or microsatellite sequences of the exon 5 of the MICA gene having SEQ ID NO: 1, SEQ ID NO: 2, SEQ ID NO: 3, SEQ ID NO: 4 and SEQ ID NO: 5.

The variants of the MICA gene can be detected by means of any method known in the state of the art, for example, but not limited to, DNA sequencing, capillary electrophoresis, mass spectrometry, single-strand conformation polymorphism (SSCP), electrochemical analysis, denaturing HPLC in electrophoresis gel, restriction fragment length polymorphism (RFLP), and hybridization analysis.

In another preferred embodiment, the first method of the invention further comprises assigning the individual of step (a) to the group of individuals having a low risk of suffering from severe sensorineural hearing loss (tonal hearing threshold greater than 40 dB) when said individual has the MICA * A4 allele detected in step (b). The sensorineural hearing loss is preferably Ménière's disease.

Steps (b) and/or (c) of the methods described above can be completely or partially automated, for example, by means of robotic sensing equipment for the detection of the amount in step (b) or the computer-aided comparison in step (c).

As it is used herein, the term "diagnosis" refers to the capacity of discriminating between individuals having a higher or lower risk of suffering from severe sensorineural hearing loss, and more preferably between individuals having a higher or lower risk of suffering from Ménière's disease with severe hearing loss.

In turn, considering the method of the present invention, other sub-classifications could be established within this main classification, which therefore facilitates choosing and establishing suitable therapeutic regimens. This discrimination as understood by a person skilled in the art is not intended to be correct in 100% of the analyzed samples. However, it requires a statistically significant amount of the analyzed samples be correctly classified. The statistically significant amount can be established by a person skilled in the art by means of using different statistical tools, as a non-limiting example, by means of determining confidence intervals, determining the significance p-value, Student's t-test or Fisher's discriminant functions, non-parametric Mann-Whitney measurements, Spearman's correlation, logistic regression, linear regression, area under the ROC curve (AUC). The confidence intervals are preferably at least 90%, at least 95%, at least 97%, at least 98% or at least 99%. The p-value is preferably less than 0.1, 0.05, 0.01, 0.005 or 0.0001. The present invention preferably allows correctly detecting predisposition to the disease or it allows differential detection of the disease in at least 60%, more preferably in at least 70%, much more preferably in at least 80%, or even much more preferably in at least 90% of the subjects of a specific group or analyzed population.

### DIAGNOSTIC KIT AND USES

Another aspect of the invention relates to a kit, hereinafter kit of the invention, comprising at least one oligonucleotide complementary to the sequence having SEQ ID NO: 1, and more preferably to the sequence having SEQ ID NO: 2. It preferably comprises two primers complementary to the sequence having SEQ ID NO: 1, and more preferably to the sequence having SEQ ID NO: 2. More preferably, the primers are capable of hybridizing with the sequence having SEQ ID NO: 1, more preferably with the sequence having SEQ ID NO: 2, under amplification conditions. In an even more preferred embodiment, the primers comprise the nucleotide sequence having SEQ ID NO: 3 and SEQ ID NO: 4, and in an even much more preferred embodiment, the nucleotide sequence primer having SEQ ID NO: 4 is preferably labeled with 6-FAM at the 5' end.

The "amplification conditions" or "elongation conditions" refer interchangeably to conditions under which a polymerase can add nucleotides to the 3' end of a polynucleotide. Said amplification or elongation conditions are very well-known in the art and are described, for example, in Sambrook and Russell, Molecular Cloning: A Laboratory Manual, 3rd Edition, 2001, Cold Spring Harbor Laboratory Press and Ausubel et al., Current Protocols in Molecular Biology, 1987-2007, John Wiley & Sons.

The term "primer" used herein is known by the person skilled in the art and refers to "oligomeric compounds", mainly "oligonucleotides", although it also refers to "modified oligonucleotides", which are capable of "priming" DNA synthesis by means of a template-dependent DNA polymerase, i.e., the 3' end of the oligonucleotide, for example, provides a free 3'-OH group to which additional "nucleotides" can be bound by a template-dependent DNA polymerase, establishing a 3'-5' phosphodiester bond in which deoxynucleoside triphosphates are used and in which pyrophosphate is released. Therefore, except for the intended function, there is no fundamental difference between a "primer", an "oligonucleotide" or a "probe" according to the invention.

According to the invention, an "oligomeric compound" is a compound consisting of "monomeric units" which can be "nucleotides" alone or "non-natural compounds" (see below), more specifically "modified nucleotides" (or "nucleotide analogues") or "non-nucleotide compounds" alone or in combinations thereof. "Oligonucleotides" and "modified oligonucleotides" (or "oligonucleotide analogues") are sub-groups of "oligomeric compounds" in the context of the invention.

In the context of the present invention, the term "oligonucleotide" refers to "polynucleotides" formed from a plurality of "nucleotides" by way of the "monomeric unit", i.e., an "oligonucleotide" belongs to specific sub-group of an "oligomeric compound" or "polymeric compound" of ribonucleic acid (RNA) or deoxyribonucleic acid (DNA) with "monomeric units". Phosphate groups are often referred to as forming the internucleoside backbone of the "oligonucleotide". The normal linkage of the RNA or DNA backbone is a 3'-5' phosphodiester bond.

The "oligonucleotides" and "modified oligonucleotides" according to the invention can be synthesized as described primarily in the art, known by the person skilled in the art. The methods for preparing oligomeric compounds having specific sequences are known in the art, and they include, among others, cloning and restriction of suitable sequences and direct chemical synthesis, for example. Chemical synthesis methods can include, for example, the phosphotriester method described by Narang S.A. et al., Methods in Enzymology 68:90-98, 1979, the phosphodiester method of Brown E.L. et al., Methods in Enzymology 68:109-151, 1979, the phosphoramidite method disclosed by Beaucage et al., Tetrahedron Letters 22:1859, 1981, and the H-phosphonate method disclosed by Garegg et al., Chem. Scr. 25:280-282, 1985, and the solid support method disclosed in US patent no. 4,458,066.

As indicated above, a "nucleic acid", as well as the "target nucleic acid", is a polymeric compound consisting of "nucleotides", as is known by the person skilled in the art. It is used herein to refer to a "nucleic acid" in a sample that must be analyzed, i.e., the presence, absence or amount of which must be analyzed in a sample.

The expression "complementary (nucleic acid) sequence of a nucleic acid sequence" as it is used herein refers to the fact that the (nucleic acid) sequence complementary to the reference sequence is the exact complement (reverse) of the nucleic acid sequence.

A polynucleotide or nucleic acid sequence can comprise the five biologically occurring bases (adenine, guanine, thymine, cytosine and uracil) and/or bases other than the five biologically occurring bases. These bases can have different purposes, for example, for stabilizing or destabilizing hybridization; for stimulating or inhibiting probe degradation; or as attachment points for detectable moieties or shielding moieties. For example, a polynucleotide of the invention can contain one or more modified, non-standard, derivatized base moieties including, but not limited to, N⁶-methyl-adenine, N⁶-tert-butyl-benzyl-adenine, imidazole, substituted imidazoles, 5-fluorouracil, 5-bromouracil, 5-chlorouracil, 5-iodouracil, hypoxanthine, xanthine, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueuosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueuosine, 5'-methoxycarboxymethyluracil, 5-methoxyuracil, 2-methylthio-N6-isopentenyladenine, uracil-5-oxyacetic acid (?), wybutosine, pseudouracil, queuosine, 2-thiocytosine, 5-methyl-2-thiouracil, 2-thiouracil, 4-thiouracil, 5-methyluracil (i.e., thymine), uracil-5-oxyacetic acid methyl ester, 3-(3-amino-3-N-2-carboxypropyl)uracil, (acp3)w, 2,6-diaminopurine, and 5-propinyl pyrimidine. Other examples of modified, non-standard, or derivatized base moieties can be found in US Patent nos. 6,001,611; 5,955,589; 5,844,106; 5,789,562; 5,750,343; 5,728,525; and 5,679,785. Furthermore, a polynucleotide or nucleic acid sequence can comprise one or more modified sugar moieties including, but not limited to, arabinose, 2-fluoroarabinose, xylulose and a hexose.

"Hybridization melting temperature" or "Tm" refers to the temperature under specific conditions at which 50% of a polynucleotide duplex is in single-stranded form and another 50% is in double-stranded form. There are readily available computer programs to calculate the Tm.

Another aspect of the invention relates to the use of the kit of the invention for the determination of the risk of suffering from sensorineural hearing loss.

In a preferred embodiment of this aspect of the invention, the sensorineural hearing loss is selected from the list consisting of: sudden hearing loss, rapidly progressive hearing loss, slowly progressive hearing loss, low-frequency sensorineural hearing loss, immune-mediated inner ear disease, or any combinations thereof.

In another preferred embodiment, the sensorineural hearing loss is furthermore associated with recurrent vertigo attacks. In an even more preferred embodiment, the MICA gene is used for obtaining useful data in the diagnosis and/or prognosis of the risk of suffering from Ménière's disease.

The terms "polynucleotide" and "nucleic acid" are used interchangeably herein, referring to the polymeric forms of nucleotides of any length, both ribonucleotides (RNA) and deoxyribonucleotides (DNA). The terms "amino acid sequence", "peptide", "oligopeptide", "polypeptide" and "protein" are used interchangeably herein and refer to a polymeric form of amino acids of any length, which can be chemically or biochemically modified, coding or noncoding amino acids.

Throughout the description and claims, the word "comprise" and variants thereof do not intend to exclude other technical features, additions, components or steps. For persons skilled in the art, other objects, advantages and features of the invention will be understood in part from the description and in part from the practice of the invention. The following examples and drawings are provided by way of illustration and not intended to be limiting of the present invention.

### DESCRIPTION OF THE DRAWINGS

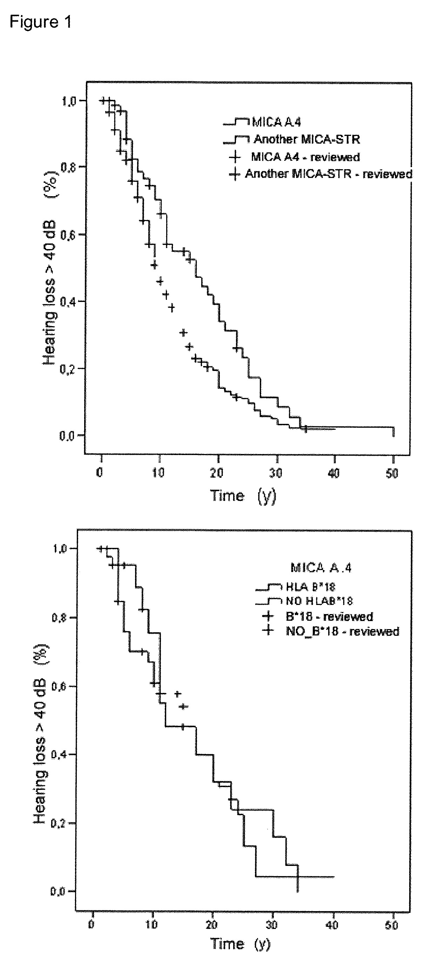

**Figure 1** **shows that MICA-STR is associated with slower hearing loss progression.** (A) Carriers of the MICA A.4 allelic variant reached stage 3 at the median of 6 years later than patients with a MICA-STR variant (log-rank test, p = 3.7x10-3). (B) Patients with the MICA*A.4-HLAB*18 haplotype or MICA A.4-with another HLAB locus did not show differences in hearing loss progression (log-rank test, p = 0.67). This demonstrates that the effect of MICA A.4 is independent of locus B.

### EXAMPLES

The invention will be illustrated below by means of tests performed by the inventors.

### Materials and Methods

### Subjects and samples

The present study included a total of 302 MD patients. Group 1 consisted of 153 patients from southeastern Spain, group 2 included 149 patients from Galicia. The samples were recruited in five centers: Hospital de Poniente de EI Ejido, Almeria; Hospital de la Fe, Valencia; Hospital Virgen de las Nieves, Granada; Hospital Clínico de Santiago de Compostela; and Complejo Hospitalario de Pontevedra between January 2007 and March 2011. The patients were diagnosed after the diagnostic scale of the American Academy of Otolaryngology-Head and Neck Surgery (AAO-HNS) (Committee on Hearing and Equilibrium guidelines for the diagnosis and evaluation of therapy in Meniere's disease. American Academy of Otolaryngology-Head and Neck Foundation, Inc. Otolaryngol Head Neck Surg 1995: 113: 181-185).

A total of 420 controls from the corresponding cities obtained from the Spanish DNA Biobank (http://www.bancoadn.org) were included as healthy controls (Table 1):

**Table 1. Baseline characteristics of cases and controls.**

| Variable | Southeastern population | | Northeastern population | |
|---|---|---|---|---|
| | MD | Control | MD | Control |
| Subjects | 153 | 276 | 149 | 144 |
| Females (%) | 53.5 | 51.4 | 52.3 | 51.4 |
| Age (years) | | | | |
| Mean | 55.2 | 38.8 | 55.1 | 37.2 |
| Median | 56 | 37 | 57.5 | 35.5 |
| Range | 15-88 | 21-64 | 12-79 | 16-64 |
| SD | 12.9 | 9.5 | 13.5 | 13.1 |

A basic neurological examination, including pure tone audiometry, nystagmus in primary position, gaze-evoked nystagmus, head-shaking nystagmus and standard caloric test, was conducted on all patients. The stage of hearing loss for each patient with definitive MD is defined as the mean of the average of four tones of 0.5, 1, 2 and 3 kHz according to the AAO-HNS's criteria: stage 1, ≤ 25 dB; stage 2, 26-40 dB; stage 3, 41-70 dB; and stage 4,> 75 dB.

The study was carried out according to the principles of Declaration of Helsinki for research with human beings. The research committee of the hospital approved the research protocol.

The genomic DNA was isolated from mononuclear peripheral blood cells with anticoagulants by means of the GenoVision M-48 robot (Qiagen, Venlo, Netherlands) and the MagAttract DNA Blood Mini M48 (192) kit of Qiagen. All patients and healthy individuals gave their informed consent for the study.

### MICA Genotyping

MICA-STR in the exon 5 of the MICA gene was analyzed in both groups using the method described by Ota *et al.* (Ota et al., 1997. Tissue Antigens: 49:448-54). The primers used for PCR were MICA 5F (SEQ ID NO: 3) 5'-CCT TTT TTT GGA CAG AAG TGC-3' and MICA 5R (SEQ ID NO: 4) 5'-CCT TAC CAT CTC CAG AAA CTG C-3°, which was labeled with 6-FAM at the 5' end. The PCR products were subjected to electrophoresis in an ABI Prism 3100XL Genetic Analyzer (Applied Biosystems, Foster City, CA, USA) and their sizes were determined using GeneMapper software version 4.0 (Applied Biosystems).

### HLA Genotyping

HLA-B * genotyping was carried out in 292 patients and 1014 controls by means of using DNA polymerase in polymerase chain reaction (PCR) with sequence-specific oligonucleotide (SSO) probes (Dynal RELI™ SSO HLA-B typing kit; Dynal Biotech, Bromborough, UK).

### Statistical Analysis

The data was analyzed using SPSS software (SPSS Inc., Chicago, IL). Locus B and MICA polymorphism allele frequencies were obtained by direct counting. Statistical analysis was performed with the X² test with Fisher's exact test, and odds ratios (OR) and 95% confidence interval (CI) were calculated to compare the frequencies observed between MD patients and controls. p-values were corrected according to the Bonferroni method for the number of comparisons made (5 for MICA alleles and 24 for HLA-B). p-values <0.05 were considered statistically significant.

MICA-STR and locus B haplotype frequencies were calculated to determine linkage disequilibrium (LD) with Haploview 4.2. software (http://www.broadinstitute.org/haploview). The linkage disequilibrium parameter (D) is calculated based on 2x2 tables. Parameter D ' corresponds to standard D. D '= 1 corresponds to total disequilibrium. The time to onset of hearing loss > 40 dB (stage 3 or 4 of hearing loss) for each locus B or MICA-STR polymorphism was determined according to the Kaplan-Meier method. Survival curves were compared by means of the log-rank test.

### Results

This study was designed to investigate the mica-STR polymorphism in DM patients. The effect of MICA was analyzed in two independent groups: group 1 included 420 individuals (153 patients, 276 controls) from southeastern Spain, group 2 included 293 individuals (149 patients, 144 controls) from Galicia to replicate the results.

The sensorineural hearing loss was stratified according to the time from onset of the disease in this series, demonstrating that the stage of hearing loss is related to the duration of MD (p=0.001).

Five MICA-STR alleles were found in the exon 5 in both groups: A6, A4, A5, A5.1 and A9. Since STR-MICA allele frequencies in the controls of subjects and populations from the Southeast to the Northeast were not significantly different (p = 0.75), all the data was grouped together for other analyses. The MICA-STR allele showing the highest frequency in both groups was A6 (34.1% and 38.2%, respectively). Unilateral and bilateral MD patients had the same allele frequency distribution and were therefore considered as a single group of patients.

Table 2 shows MICA-STR allele frequencies in MD patients and controls. It was found that the MICA * A5 allele of the microsatellite is more common in controls (12.7%) than in MD patients (6.9%) in the group from the Southeast (PC = 0.04, OR = 0.51 [95% CI, 0.30-0.84]). However, the inventors were unable to replicate these findings in the group from the Northeast, and a meta-analysis of the two groups showed that MICA*A5 is not associated with MD.

**Table 2. MICA-STR allele frequency in MD patients and controls.**

| **Ménière's disease** | Controls | MD | OR (95% CI) | p-value | Corrected |
|---|---|---|---|---|---|
| Southeast | N=276 | N=153 | | | |
| A.4 | 82 (14.9) | 46(15.0) | 1.01 (0.68-1.50) | 0.95 | NS |
| A.5 | 70 (12.7) | 21 (6.9) | 0.51 (0.30-0.84) | 0.008 | 0.04 |
| A.5.1 | 141 (25.6) | 72 (23.5) | 0,86 (0.65-1.24) | 0.50 | NS |
| A.6 | 188 (34.1) | 121 (39.5) | 1.26 (0.95-1.69) | 0.11 | NS |
| A.9 | 70 (12.7) | 46 (15.0) | 1.22 (0.81-1.82) | 0.34 | NS |
| | | | | | |
| Northeast | N=144 | N=149 | | | |
| A.4 | 36 (12.5) | 29 (9.7) | 0.76 (0.45-1.27) | 0.29 | NS |
| A.5 | 38 (13.2) | 35(11.7) | 0.88 (0.54-1.43) | 0.60 | NS |
| A.5.1 * | 68 (23.6) | 82(27.5) | 1.23 (0.85-1.78) | 0.28 | NS |
| A.6 | 110 (38.2) | 111 (37.2) | 0.96 (0.69-1.34) | 0.81 | NS |
| A.9 | 36 (12.5) | 41 (13.8) | 1.12 (0.69-1.81) | 0.65 | NS |
| | | | | | |

| **Total Ménière's disease** | Controls N=420 | MD N=302 | OR (95% CI) | p-value | Corrected p-value |
|---|---|---|---|---|---|
| A.4 | 118 (12.4) | 75 (12.4) | 0.86 (0.64-1.18) | 0.36 | NS |
| A.5 | 108 (12.9) | 56 (12.9) | 0.69 (0.49-0.97) | 0.03 | NS |
| A.5.1 | 209 (24.9) | 154 (25.5) | 1.03 (0.81-1.31) | 0.80 | NS |
| A.6 | 298 (35.5) | 232 (38.4) | 1.13 (0.91-1.41) | 0.26 | NS |
| A.9 | 106 (12.6) | 87 (14.4) | 1.16 (0.86-1.58) | 0.33 | NS |

HLA-B allele frequency distribution was no different between the northeastern group and the southeastern group and all the data was grouped together. In addition, HLA typing did not find HLA-B * to have a significant association with MD (Table 3).

**Table 3. HLA locus B frequency in MD patients and controls.**

| Locus | Unilateral MD | Bilateral MD | OR | p-value | Cases (n=292) | Controls (n=1014) | OR | p-value |
|---|---|---|---|---|---|---|---|---|
| B*07 | .072 | .072 | 1.002 | .995 | .072 | .092 | 0.762 | .126 |
| B*08 | .049 | .048 | 1.026 | .949 | .046 | .052 | 0.878 | .557 |
| B*13 | .013 | .008 | 1.648 | .695 | .010 | .019 | 0.529 | .142 |
| B*14 | .092 | .040 | 2.426 | .016 | .067 | .051 | 1.340 | .134 |
| B*15 | .036 | .056 | 0.634 | .260 | .045 | .053 | 0.836 | .423 |
| B*18 | .066 | .064 | 1.0026 | .940 | .067 | .089 | 0.730 | .084 |
| B*27 | .020 | .052 | 0.366 | .037 | .033 | .037 | 0.875 | .610 |
| B*35 | .095 | .060 | 1.646 | .128 | .084 | .103 | 0.797 | .171 |
| B*37 | .023 | .012 | 1.934 | .524 | .017 | .010 | 1.664 | .184 |
| B*38 | .033 | .024 | 1.379 | .538 | .029 | .028 | 1.055 | .848 |
| B*39 | .013 | .020 | 0.651 | .738 | .017 | .014 | 1.244 | .556 |
| B*40 | .039 | .036 | 1.097 | .837 | .039 | .034 | 1.181 | .498 |
| B*41 | .023 | .020 | 1.151 | .812 | .021 | .013 | 1.554 | .205 |
| B*44 | .167 | .204 | 0.783 | .266 | .182 | .151 | 1.247 | .074 |
| B*45 | .008 | .016 | 0.406 | .417 | .010 | .016 | 0.627 | .292 |
| B*49 | .049 | .040 | 1.241 | .604 | .048 | .031 | 1.570 | .050 |
| B*50 | .030 | .036 | .814 | .668 | .031 | .030 | 1.043 | .880 |
| B*51 | .095 | .108 | 0.868 | .615 | .103 | .090 | 1.154 | .361 |
| B*52 | .016 | .024 | 0.678 | .555 | .019 | .015 | 1.236 | .549 |
| B*53 | .016 | .012 | 1.372 | .735 | .014 | .016 | 0.866 | .717 |
| B*55 | .007 | .004 | 1.644 | 1.000 | .007 | .010 | 0.659 | .443 |
| B*56 | .003 | ... | ... | 1.000 | .002 | .002 | 0.694 | 1.00 |
| B*57 | .023 | .032 | 0.711 | .513 | .027 | .025 | 1.091 | .763 |
| B*58 | .013 | .012 | 1.094 | 1.000 | .012 | .013 | 0.899 | .802 |

Linkage disequilibrium calculations were made in controls and patients having HLA-B-MICA-STR haplotypes with a frequency ≥ 1.5%. As was expected from the chromosomal position of MICA and HLA-B *, there was significant linkage disequilibrium between certain HLA-B * and STR-MICA alleles. Thirteen (13) haplotypes were found in the control group, most of the common property being MICA * A6-HLA-B * 44 (7.2%), MICA * A6-HLA-B * 51 (7.4%) and MICA * A5.1-HLA-B * 7 (6.5%). The same frequency was found in MD patients, and there were no differences in the MICA-STR and HLA-B haplotypes between cases and controls (Table 4).

| | ***Control Group*** | | | | ***Group of Patients*** | | | |
|---|---|---|---|---|---|---|---|---|
| Haplotype | HF | D' | LOD | Pc | HF | D' | LOD | Pc |
| MICA*A.4-B*18 | 0.053 | 0.906 | 36.22 | 1.18x10⁻²⁴ | 0.042 | 0.829 | 17.51 | 2.64x10⁻¹³ |
| MICA*A.4-B*27 | 0.026 | 1.000 | 24.32 | 5.28x10⁻⁹ | 0.023 | 0.864 | 9.35 | 3.30x10⁻⁷ |
| MICA*A.5-B*15 | 0.026 | 0.603 | 11.10 | 2.15x10⁻⁷ | 0.023 | 0.390 | 3.56 | 1.36x10⁻⁵ |
| MICA*A.5-B*35 | 0.025 | 0.201 | 2.21 | 0.02 | 0.026 | 0.100 | 0.44 | 5.28x10⁻⁴ |
| MICA*A.5.1-B*7 | 0.065 | 0.764 | 22.02 | 2.02x10⁻¹³ | 0.055 | 0.865 | 10.14 | 4.62x10⁻¹⁰ |
| MICA*A.5.1-B*8 | 0.029 | 0.679 | 6.92 | 5.94x10⁻⁶ | 0.036 | 0.851 | 5.97 | 2.42x10⁻⁷ |
| MICA*A.5.1-B*35 | 0.015 | 1.000 | 4.07 | 0.01 | 0.015 | 0.002 | 0.00 | NS |
| MICA*A.5.1-B*40 | 0.018 | 0.202 | 0.56 | NS | 0.023 | 0.283 | 0.61 | 0.06 |
| MICA*A.6-B*44 | 0.072 | 0.442 | 4.69 | 6.16x10⁻⁵ | 0.098 | 0.332 | 2.47 | 0.02 |
| MICA*A.6-B*49 | 0.024 | 0.908 | 5.11 | 7.26x10⁻⁶ | 0.034 | 0.825 | 3.89 | 6.6x10⁻⁴ |
| MICA*A.6-B*50 | 0.025 | 1.000 | 6.02 | 2.86x10⁻⁶ | 0.028 | 0.657 | 2.01 | 0.02 |
| MICA*A.6-B*51 | 0.074 | 0.865 | 15.54 | 1.87x10⁻¹⁴ | 0.089 | 0.828 | 8.36 | 3.52x10⁻¹⁴ |
| MICA*A.9-B*35 | 0,024 | 0.340 | 6.85 | 0.02 | 0.019 | 0.353 | 3.53 | NS |
| MICA*A.9-B*57 | 0.023 | 0.878 | 9.70 | 6.03x10⁻¹⁵ | 0.021 | 0.896 | 5.59 | 2.86x10⁻⁷ |

Table 4. The haplotype frequency (HF) and linkage disequilibrium (LD), analysis of HLA-B and STR-MICA alleles. The LD analysis was limited to HLA-BMICA-STR haplotypes with frequencies ≥ 1.5% observed in controls.

Data on hearing loss was available for all ED patients. Seventeen patients were in stage 1 (6.2%), 60 in stage 2 (19.7%), 157 in stage 3 (51.7%) and 68 in stage 4 (22.3%). The median time for developing hearing loss > 40 dB is 16 years (95% confidence interval, 9.23) for patients with the MICA * A.4 allele, and 10 years (9-11) for patients with another MICA-STR allele (log-rank test, p = 0.0038; Figure 1).

### Conclusions

Ménière's disease is a multifactorial disease and genetic, epigenetic and environmental factors contribute to its manifestation. Although various associations of HLA class I and II with MD have been reported in case-control studies through different ethnic groups, these finds have not been replicated in different populations. In addition, although various reasons can explain that the replication studies are unable to confirm the initially observed association (selection bias, population stratification or specific ethnic variants), HLA association studies require a large sample size since the number of alleles for comparing locus B or DR is very high. The data of the present application includes the most extensive series for studying HLA-B in ED patients and confirming the absence of association of MD with locus B.

This lack of HLA gene replication means that the present study is the first to analyze the association of genes located in this region with polymorphic microsatellites, such as MICA-STR, which has demonstrated linkage disequilibrium with HLA-B genes.

STR-MICA allele frequency in MD patients and controls in Spain was studied. There were no differences in MICA-STR distribution in controls from Galicia or the Mediterranean population, suggesting that this polymorphism is homogenous within the Spanish population. However, it has been found that MICA * A5 was more common in controls than in patients in the Mediterranean cohort, suggesting differences with the Galician group. The epigenetic and environmental factors triggering an MD attack can explain these findings.

The MICA * A5 allele has been associated with ulcerative colitis, with poor progression of type I diabetes mellitus and multiple sclerosis.

MICA * A5 shows linkage disequilibrium with HLA-B * 15 and HLA-B * 35. Since the HLA-B * 15 allele frequencies were not different between cases and controls and the HLA-B * 35 allele frequencies were 5% and 10%, respectively, the MICA * A5-HLA-B35 haplotype can possibly be a protective factor for MD. When comparing MICA-HLA-B haplotype frequencies between patients and controls, there were no statistically significant differences. However, the MICA * A4 allele was associated with a longer time for developing hearing loss > 40 dB (16 years compared to 10 years for any other MICA allele). This suggests that interaction between this allele and the NKGD receptor can determine less damage to inner ear tissues.

Although the importance of high/low NKG2D receptor affinity in terms of immune activation it is not completely proven, the presence of repeats in exon 5 of the MICA gene may influence interaction with this receptor, modify NK cells, γδ T-cells and αβ CD8+ T-cells, and allow an exacerbated immune response. However, the responses of NK cells and CD8+ T-cells have not been investigated in MD.

## Claims

1. Use of the MICA gene for obtaining useful data in the diagnosis and/or prognosis of sensorineural hearing loss.

2. Use of the MICA gene according to the preceding claim, where the sensorineural hearing loss is selected from the list consisting of: sudden hearing loss, rapidly progressive hearing loss, slowly progressive hearing loss, low-frequency sensorineural hearing loss, immune-mediated inner ear disease, or any combinations thereof.

3. Use of the MICA gene according to any of claims 1-2, where the sensorineural hearing loss is furthermore associated with recurrent vertigo attacks.

4. Use of the MICA gene according to any of claims 1-3 in the diagnosis and/or prognosis of Ménière's disease.

5. Use of the MICA gene according to any of claims 1-4, **characterized in that** the region of the MICA gene used is the 5' region of the terminal exon (SEQ ID NO: 2).

6. A method for obtaining useful data for the diagnosis and/or prognosis of sensorineural hearing loss, which comprises:
a) obtaining genomic DNA from a biological sample isolated from an individual, and
b) detecting variants of the MICA gene in the genomic DNA of the biological sample isolated in (a).

7. The method for obtaining useful data according to the preceding claim, which further comprises:
c) comparing the variants detected in step (b) with a reference variant.

8. The method for obtaining useful data according to any of claims 6-7, **characterized in that** the region of the MICA gene used is the 5' region of the terminal exon (SEQ ID NO: 2).

9. The method for obtaining useful data according to any of claims 6-8, where the isolated sample is peripheral blood DNA.

10. The method for obtaining useful data according to any of claims 6-9, which further comprises assigning the individual of step (a) to the group of individuals with a low risk of suffering from severe sensorineural hearing loss when said individual has the MICA * A4 allele.

11. The method for obtaining useful data according to any of claims 6-10, where the sensorineural hearing loss is selected from the list consisting of: sudden hearing loss, rapidly progressive hearing loss, slowly progressive hearing loss, low-frequency sensorineural hearing loss, immune-mediated inner ear disease, or any combinations thereof.

12. The method for obtaining useful data according to any of claims 6-11, where the sensorineural hearing loss is furthermore associated with recurrent vertigo attacks.

13. Use of the method for obtaining useful data according to any of claims 6-12 in the diagnosis and/or prognosis of Ménière's disease.

14. A kit comprising at least one oligonucleotide complementary to the sequence having SEQ ID NO: 1.

15. The kit according to the preceding claim, comprising two primers complementary to the sequence having SEQ ID NO: 1, capable of hybridizing with said sequence or with its complementary sequence under amplification conditions.

16. The kit according to any of claims 14-15, where the primers are capable of hybridizing with the sequence having SEQ ID NO: 2 or with its complementary sequence.

17. The kit according to any of claims 14-16, where the primers are the nucleotide sequence having SEQ ID NO: 3 and SEQ ID NO: 4, respectively.

18. The kit according to claim 17, where the nucleotide sequence primer having SEQ ID NO: 4 is labeled with 6-FAM at the 5' end.

19. Use of the kit according to any of claims 12-15 for the diagnosis and/or prognosis of sensorineural hearing loss.

20. Use of the kit according to the preceding claim, where the sensorineural hearing loss is selected from the list consisting of: sudden hearing loss, rapidly progressive hearing loss, slowly progressive hearing loss, low-frequency sensorineural hearing loss, immune-mediated inner ear disease, or any combinations thereof.

21. Use of the kit according to any of claims 19-20, where the sensorineural hearing loss is furthermore associated with recurrent vertigo attacks.

22. Use of the kit according to any of claims 19-21 in the diagnosis and/or prognosis of Ménière's disease.


LISTADO DE SECUENCIAS

<110>  Fundación Pública Andaluza Progreso y Salud
<110>  Agencia Publica Empresarial Hospital de Poniente
<110>  Servicio Andaluz de Salud
<110>  Servizo Galego de Saúde
<110>  Fundación para la Investigación del Hospital Universitario
       La Fe

<120>  Método de obtención de datos útiles para el diagnóstico y
       pronóstico de la hipoacusia neurosensorial

<130>  PCT-05972

<160>  9

<170>  PatentIn version 3.5

<210>  1
<211>  1348
<212>  DNA
<213>  Homo sapiens

<400>  1
tttctctttc ttttccggac cctgcagtgg cgcctaaagt ctgagagagg gaagtcgcct      60

ctgtgctcgt gagtgcatgg ggtataagag ccccacagtc ttcgttataa cctcacggtg     120

ctgtcctggg atggatctgt gcagtcaggg tttcttgctg aggtacatct ggatggtcag     180

cccttcctgc gctatgacag gcagaaatgc agggcaaagc cccagggaca gtgggcagaa     240

gatgtcctgg gaaataagac atgggacaga gagaccaggg acttgacagg gaacggaaag     300

gacctcagga tgaccctggc tcatatcaag gaccagaaag aaggcttgca ttccctccag     360

gagattaggg tctgtgagat ccatgaagac aacagcacca ggagctccca gcatttctac     420

tacgatgggg agctcttcct ctcccaaaac ctggagactg aggaatggac agtgccccag     480

tcctccagag ctcagacctt ggccatgaac gtcaggaatt tcttgaagga agatgccatg     540

aagaccaaga cacactatca cgctatgcat gcagactgcc tgcaggaact acggcgatat     600

ctagaatccg gcgtagtcct gaggagaaca gtgcccccca tggtgaatgt cacccgcagc     660

gaggcctcag agggcaacat caccgtgaca tgcagggctt ccagcttcta tccccggaat     720

atcatactga cctggcgtca ggatggggta tctttgagcc acgacaccca gcagtggggg     780

gatgtcctgc ctgatgggaa tggaacctac cagacctggg tggccaccag gatttgccga     840

ggagaggagc agaggttcac ctgctacatg gaacacagcg ggaatcacag cactcaccct     900

gtgccctctg ggaaagtgct ggtgcttcag agtcattggc agacattcca tgtttctgct     960

gttgctgctg gctgctgcta tttttgttat tattattttc tatgtccgtt gttgtaagaa    1020

gaaaacatca gctgcagagg gtccagagct cgtgagcctg caggtcctgg atcaacaccc    1080

agttgggacg agtgaccaca gggatgccac acagctcgga tttcagcctc tgatgtcagc    1140

tcttgggtcc actggctcca ctgagggcac ctagactcta cagccaggcg gctggaattg    1200

aattccctgc ctggatctca caagcacttt ccctcttggt gcctcagttt cctgacctat 1260

gaaacagaga aaataaaagc acttatttat tgttgttgga ggctgcaaaa tgttagtaga 1320

tatgaggcat ttgcagctgt gccatatt 1348

<210> 2
<211> 63
<212> DNA
<213> Homo sapiens

<400> 2
acattccatg tttctgctgt tgctgctggc tgctgctatt tttgttatta ttattttcta 60

tgt 63

<210> 3
<211> 21
<212> DNA
<213> Homo sapiens

<400> 3
cctttttttg gacagaagtg c 21

<210> 4
<211> 21
<212> DNA
<213> Homo sapiens

<400> 4
ccttaccatc tccagaaact g 21

<210> 5
<211> 12
<212> DNA
<213> Homo sapiens

<400> 5
gctgctgctg ct 12

<210> 6
<211> 15
<212> DNA
<213> Homo sapiens

<400> 6
gctgctgctg ctgct 15

<210> 7
<211> 17
<212> DNA
<213> Homo sapiens

<400> 7
gctgctggct gctgctg 17

<210>  8
<211>  18
<212>  DNA
<213>  Homo sapiens

<400>  8
gctgctgctg ctgctgct                                                    18


<210>  9
<211>  27
<212>  DNA
<213>  Homo sapiens

<400>  9
gctgctgctg ctgctgctgc tgctgct                                          27